# EUROPÄISCHE PATENTANMELDUNG

(11) **EP 3 603 617 A1**
(43) Veröffentlichungstag der Anmeldung: **05.02.2020**
(21) Anmeldenummer: 18186429.9
(22) Anmeldetag: 30.07.2018
(51) Int. Cl.: A61K 9/00, A61K 31/7088, A61P 11/06, A61M 11/00

(54) **AEROSOLERZEUGUNGSEINRICHTUNG ZUR INHALATIVEN VERABREICHUNG EINER ANTISENSE-MOLEKÜL-HALTIGEN ZUSAMMENSETZUNG**

(71) Anmelder: Sterna Biologicals GmbH & Co. KG, 35037 Marburg (DE)
(72) Erfinder: Renz, Jonas, 81675 München (DE)
(74) Vertreter: Patentanwälte Olbricht Buchhold Keulertz

(57) **Zusammenfassung**

Verwendung einer Aerosolerzeugungseinrichtung zur Erzeugung eines Aerosols einer Zusammensetzung, zur inhalativen Verabreichung der Zusammensetzung, wobei die Zusammensetzung mindestens ein Antisense-Molekül umfasst, wobei die Aerosolerzeugungseinrichtung eine Membran mit einer Perforation aufweist, wobei die Zusammensetzung zur Erzeugung des Aerosols mit der Membran in Kontakt gebracht wird, und die Membran der Aerosolerzeugungseinrichtung in Vibration versetzt wird, wobei die Perforation von mindestens einer Gruppe von Durchgangsöffnungen gebildet ist, wobei jede Durchgangsöffnung einer Gruppe an ihrer engsten Stelle einen Durchmesser von mindestens 3 µm und höchstens 5 µm aufweist und wobei die mindestens eine Gruppe von Durchgangsöffnungen maximal 500 Durchgangsöffnungen pro mm² der Membran umfasst.

## Beschreibung

Die Erfindung betrifft die Verwendung einer Aerosolerzeugungseinrichtung zur Erzeugung eines Aerosols einer Antisense-Molekül-haltigen Zusammensetzung, zur inhalativen Verabreichung der Zusammensetzung. Die Erfindung betrifft insbesondere die Verwendung nach Anspruch 1.

Falls nicht anders angegeben, werden die Begriffe "Expression" und "Genexpression" im Folgenden synonym verwendet. Darüber hinaus bezeichnet der Begriff "Aerosol" im Folgenden ein Gemisch aus festen oder flüssigen Partikeln in Gasen oder Luft, wohingegen der Begriff "Nebel" fein verteilte Flüssigkeitstropfen in der Luft bezeichnet.

Entzündungen der Atemwege sind die am häufigsten auftretenden Atemwegserkrankungen. Sie führen oft zu chronischen Entzündungen. Ein Beispiel für solche Entzündungen stellen Allergien dar, die durch eine überschießende Immunantwort auf für den Menschen üblicherweise harmlose Antigene wie Pollen, Tierhaare, Nahrungsmittel, Milben, Konservierungsstoffe, Farbstoffe, Reinigungsmittel etc. hervorgerufen werden. Andere typische Atemwegserkrankungen, die zu chronischen Entzündungen führen, sind zum Beispiel Autoimmunerkrankungen wie Asthma oder chronisch-obstruktive Lungenerkrankungen (COPD). Beim Fortschreiten der initialen Entzündungsreaktion bis zur chronischen Entzündung sind Destruktions- und Umbauprozesse beteiligt. In der Folge geht die Toleranz verloren, also die Fähigkeit, körpereigene Zellen, kommensale Erreger oder belanglose Allergene nicht anzugreifen.

An der Immunantwort sind Komponenten des angeborenen sowie des erworbenen Immunsystems beteiligt. Hierbei spielen vor allem der Th1-zellspezifische Transkriptionsfaktor Tbet, der die spezifische Entwicklung von Th1-Zellen reguliert, und der Th2-zellspezifische Transkriptionsfaktor GATA-3, der die spezifische Entwicklung von Th2-Zellen reguliert, eine wichtige Rolle.

Im gesunden Körper besteht ein Gleichgewicht zwischen Th1- und Th2-Zellen. Dieses Gleichgewicht kann durch bestimmte Einflüsse gestört werden. So ist zum Beispiel bei einer chronischen Entzündung das Gleichgewicht zwischen Th1- und Th2-Zellen durch die Expression von GATA-3 bzw. durch die Hemmung von Tbet zugunsten der Th2-Zellen verschoben. Es kommt zu einer Dominanz der Th2-Zellen, die wiederum typisch für viele chronisch entzündliche Erkrankungen in der Spätphase ist.

In anderen Fällen liegt eine Dominanz der Th1-Zellen vor, wenn es zu einer Hemmung von GATA-3 kommt bzw. die Expression von Tbet für ein steigendes Th1-Zellen-Niveau sorgt.

Eine Therapiemethode besteht nun darin, die Transkriptionsfaktoren GATA-3 bzw. Tbet spezifisch zu hemmen bzw. "auszuschalten", sobald eine Dominanz von Th1-bzw. Th2-Zellen vorliegt. Auf diese Weise lässt sich das Gleichgewicht zwischen den beiden Zelltypen wiederherstellen. Eine Möglichkeit stellt dabei die gezielte Inhibition der Genexpression der Transkriptionsfaktoren GATA-3 bzw. Tbet dar, etwa durch die Verwendung geeigneter Antisense-Moleküle.

In der WO 2005/033314 A2 ist die Wirkweise verschiedener GATA-3-spezifischer bzw. Tbet-spezifischer Antisense-Moleküle in Form von DNAzymen beschrieben. Der Offenbarungsgehalt der WO 2005/033314 wird daher als technologischer Hintergrund der vorliegenden Erfindung angesehen.

Insbesondere im Falle von Atemwegserkrankungen werden die jeweiligen Wirkstoffe inhalativ verabreicht, wobei unter "Inhalation" das Einatmen von Aerosolen oder gasförmigen Wirkstoffen bezeichnet wird. Die Inhalation ist eine bewährte Methode und ermöglicht den direkten Zugang zu verschiedenen Regionen der Lunge. Auf diese Weise ist eine intra- und transpulmonale Medikamentenapplikation der Wirkstoffe möglich. Die Möglichkeit, über die Inhalation ein Arzneimittel über die Lunge bzw. über die Zielzellen in der Lunge aufzunehmen, kann einen wichtigen Faktor für die Wirksamkeit eines Arzneimittels darstellen. Hierbei spielt unter anderem die Größe der zu inhalierenden Partikel eine Rolle. Sie wird über den MMD ("Mass Median Diameter") bzw. über den MMAD ("Mass Median Aerodynamic Diameter") charakterisiert.

Es sind zahlreiche Inhalationsgeräte bekannt, wobei üblicherweise drei Gerätetypen eingesetzt werden, die sich jeweils in ihrer Handhabung unterscheiden: Dosierinhalatoren, Pulverinhalatoren sowie insbesondere Aerosolerzeugungseinrichtungen. Prinzipiell gilt dabei, dass Tischgeräte nachteilig sind, weil bei ihrer Verwendung keine gezielte Verneblung in die Lunge erfolgt. Vielmehr hängt es vom Atemmanöver des Patienten ab, wie viele Tröpfchen er einatmet.

Aerosolerzeugungseinrichtungen, auch als Vernebler bezeichnet, trennen feine Tröpfchen (im Folgenden auch als Tropfen oder Partikel bezeichnet) von einem flüssigen Reservoir oder einer Lösung ab. Dadurch entsteht ein Aerosol, das vom Patienten eingeatmet werden kann. Es sind drei Funktionsprinzipien bekannt, nach denen die Vernebler eingeteilt werden. Bei Düsenverneblern erzeugt ein starker Luftstrom an einer Düse einen Unterdruck und zieht so Tröpfchen aus einem kapillaren System. Weil die Tröpfchen unterschiedliche Größen aufweisen, wird eine Prallplatte eingesetzt, um zu große Tröpfchen zurückzuhalten.

Bei Ultraschallverneblern werden die Tröpfchen durch einen Ultraschall generiert. Je höher die Frequenz des Ultraschalls, desto feiner werden die Tröpfchen.

Membranvernebler zeichnen sich durch eine sehr dünne Membran mit einer Vielzahl an Mikrobohrungen oder Durchgangsöffnungen aus, wobei eine Membran weit über tausend solcher Durchgangsöffnung aufweisen kann. Die Membran wird zur Vibration gebracht, wodurch jede Durchgangsöffnung als Pumpe fungiert und Tröpfchen mit einem definierten MMAD produziert.

Unter Antisense-Molekülen werden in der Regel Moleküle verstanden, die entweder aus meist einzelsträngigen RNA- und/oder DNA-Oligonukleotiden aufgebaut sind oder zumindest einen solche Oligonukleotid-Bestandteil aufweisen. Oligonukleotide sind wiederum aus wenigen Nukleotiden aufgebaute Moleküle, wie zum Beispiel Primer, die in der Polymerase-Kettenreaktion (PCR) eingesetzt werden. Antisense-Moleküle besitzen meist Oligonukleotide, deren Basensequenz komplementär zu einer zellulären, viralen oder synthetischen RNA- oder DNA-Molekülen ist und über Watson-Crick-Basenpaarung an diese Moleküle binden können. Dieser Umstand kann zum Beispiel dazu genutzt werden, die Funktion einer beliebigen Ziel-mRNA zu hemmen, etwa durch die spezifische Bindung der Antisense-Moleküle an die Ziel-mRNA. Sowohl in der Forschung als auch in der therapeutischen Anwendung werden. Antisense-Moleküle eingesetzt, um die Expression eines Gens gezielt abgeschwächt beziehungsweise gänzlich zu unterdrücken. Seit einigen Jahren sind auch Antisense-Moleküle bekannt, die nicht auf Nukleinsäurebasis aufgebaut sind. Derartige Moleküle weisen dann in aller Regel Nukleinsäure-Analoga auf, welche hinsichtlich ihrer Fähigkeit der Basenpaarung mit echten Nukleinsäuren übereinstimmen.

Zurzeit sind für Antisense-Molekül-haltige Zusammensetzungen keine geeigneten Rahmenbedingungen oder Parameter für die Verwendung einer solchen Zusammensetzung mittels einer Aerosolerzeugungseinrichtung zur inhalativen Behandlung eines an einer mit chronischen Entzündungen einhergehenden Atemwegserkrankung leidenden Patienten bekannt. Vielmehr lässt sich bei der Verwendung einer Antisense-Molekül-haltigen Zusammensetzung das Phänomen beobachten, dass die Membranen der jeweils verwendeten Aerosolerzeugungseinrichtungen nach kurzer Zeit verstopfen können, weil Antisense-Molekül-haltige Zusammensetzungen oder Lösungen hoch viskos sind.

Der Erfindung liegt daher die Aufgabe zugrunde, eine Verwendung einer Aerosolerzeugungseinrichtung zu ermöglichen, mit der sich eine Antisense-Molekül-haltige Zusammensetzung zur Behandlung eines an einer mit chronischen Entzündungen einhergehenden Atemwegserkrankung leidenden Patienten inhalativ verabreichen lässt. Insbesondere soll die Antisense-Molekül-haltige Zusammensetzung mithilfe der Aerosolerzeugungseinrichtung einfach, zuverlässig und reproduzierbar applizierbar sowie definiert zu zerstäuben sein.

Diese Aufgabe wird erfindungsgemäß durch Verwendung einer Aerosolerzeugungseinrichtung zur Erzeugung eines Aerosols und einer entsprechend angepassten Zusammensetzung zur inhalativen Verabreichung gelöst, wobei die Zusammensetzung mindestens ein Antisense-Molekül umfasst, wobei die Zusammensetzung zur Erzeugung des Aerosols mit der Membran in Kontakt gebracht wird und die Membran der Aerosolerzeugungseinrichtung in Vibration versetzt wird, wobei die Perforation von mindestens einer Gruppe von Durchgangsöffnungen gebildet ist, wobei jede Durchgangsöffnung einer Gruppe an ihrer engsten Stelle einen Durchmesser von mindestens 3 µm und höchstens 5 µm aufweist und wobei die mindestens eine Gruppe von Durchgangsöffnungen maximal 500 Durchgangsöffnungen pro mm² der Membran umfasst. Die Antisense-Molekül-haltige Zusammensetzung ist insbesondere eine Flüssigkeit oder eine Lösung. Vorzugsweise ist vorgesehen, dass jede Durchgangsöffnung der Membran an ihrer engsten Stelle einen Durchmesser von mindestens 3 µm und höchstens 5 µm aufweist. Die Durchgangsöffnung bzw. Mikrobohrungen können dabei verschiedene Geometrien aufweisen. Sie können zum Beispiel schlitzförmig, eckig oder oval sein oder eine Lochgeometrie aufweisen. Vorliegend werden vor allem Löcher als Mikrobohrungen der Membran eingesetzt, wobei mindestens 100 Mikrobohrungen oder Durchgangsöffnungen pro Membran vorgesehen sind. Ferner wird die Membran durch ein piezoelektrisches Element angeregt und in Schwingungen versetzt. Das Antisense-Molekül ist ausgewählt aus der Gruppe umfassend DNAzyme, siRNAs, asDNAs oder Ribozyme. Bei der vorliegenden Anmeldung werden bevorzugt DNAzyme als Antisense-Moleküle eingesetzt. Wahlweise kann das Antisense-Molekül jedoch auch aus der Gruppe der Moleküle umfassend Phosphorothioat-Oligonukleotide (PTO), 2'-Fluoro-RNAs, FANAs, 2-O-Methyl-Oligonukleotide, 2'-O-Methoxyethyl-Oligonukleotide, "constrained ethyl oligonucleotides" (cEt), "locked nucleic acids" (LNA), µ2'-O,4'-C-ethylene-bridged nucleic acid" (ENA), Peptidnukleinsäuren (PNA), Morpholino, Ugimere, Tricyclo-DNA, DNA-Primer oder Aptamere.

Ein wesentlicher Vorteil, der sich aus der erfindungsgemäßen Verwendung der Aerosolerzeugungseinrichtung ergibt, besteht darin, dass sich die Antisense-Molekül-haltige Zusammensetzung in ein Aerosol überführen lässt, dessen Partikel einen VMD MV ("Volume Median Diameter") von ca. 4 µm aufweisen. Nur ein Aerosol mit Partikeln kleiner als 5 µm kann, aufgrund von Impaktationskräften von größeren Partikeln, chronische Entzündungen in den mittleren und unteren Atemwegen erreichen, sodass entsprechende Antisense-Moleküle, insbesondere DNAzyme, den Wirkort erreichen und dort therapeutisch wirksam werden. Somit steht eine wirksame, einfache und sichere Verabreichungsmöglichkeit der viskosen Antisense-Molekül-haltigen Zusammensetzung durch Inhalation zur Verfügung.

Mit der erfindungsgemäßen Verwendung einer Aerosolerzeugungseinrichtung mit einer vibrierenden Membran, deren Mikrobohrungen jeweils einen Durchmesser von 3 µm bis 5 µm aufweisen, ist es möglich, Antisense-Molekül-haltige Zusammensetzungen, die in der Regel hoch viskos sind, zuverlässig in ein therapeutisch wirksames und inhalierbares Aerosol zu überführen. Die erfindungsgemäße Verwendung erlaubt es dem Patienten außerdem, pro Aktivierungsereignis der Aerosolerzeugungseinrichtung bzw. pro Inhalationsphase stets eine definierte Wirkstoffdosis zu erhalten. Somit kann eine Antisense-Molekül-haltigen Zusammensetzung sicher, zuverlässig und reproduzierbar verabreicht werden.

Das Merkmal, wonach die Gruppe von Durchgangsöffnungen maximal 500 Durchgangsöffnungen pro mm² der Membran umfasst, unterstützt den vorteilhaften Effekt, den die Durchgangsöffnungen mit einem Durchmesser von mindestens 3 µm und höchstens 5 µm auf die Tröpfchenbildung haben, nämlich, dass sich die Antisense-Molekül-haltige Zusammensetzung in ein Aerosol überführen lässt, dessen Partikel einen VMD MV von ca. 4 µm aufweisen. So können Membranen mit einer höheren Lochdichte als 500 Durchgangsöffnungen pro mm² dazu führen, dass die entstehenden Tröpfchen oder Partikel unmittelbar nach Austritt aus den Durchgangsöffnungen der Membran zu größeren Partikeln verschmelzen, aggregieren oder vereinigen. Das hätte wiederum zum Nachteil, dass die Partikel oder Tropfen aufgrund von Impaktationskräften nicht oder zumindest in verminderter Weise an ihren entsprechenden Wirkort gelangen würden. Folglich könnten die Antisense-Moleküle bzw. insbesondere die DNAzyme ihre therapierende Wirkung nicht an dem Ort entfalten, an dem die chronische Entzündung vorliegt. Demgegenüber steht durch die erfindungsgemäße Verwendung von Aerosolerzeugungseinrichtungen mit Membranen, die maximal 500 Durchgangsöffnungen pro mm² umfassen, eine wirksame, einfache und sichere Verabreichungsmöglichkeit der viskosen Antisense-Molekül-haltigen Zusammensetzung durch Inhalation zur Verfügung. Die Zerstäubung der Zusammensetzung in ein Aerosol mit Tröpfchen der bevorzugten Größe wird außerdem durch die Vibration der Membran verstärkt.

Die Aerosolerzeugungseinrichtung ist dabei bevorzugt ein tragbares und handliches Gerät, das auch in Notfallsituationen einsetzbar ist. Die Energieversorgung der Vorrichtung erfolgt dabei über Kabel, über eine oder mehrere Batterien oder induktiv. Durch die erfindungsgemäße Verwendung ist das erzeugte Aerosol der Antisense-Molekül-haltigen Zusammensetzung außerdem frei von schädlichen Treibgasen.

Die aufgeführte Verwendung verringert die Risiken für Nebenwirkungen im restlichen Organismus, zudem ist durch das örtlich gezielte Wirken der Antisense-Moleküle eine geringere Wirkstoffdosis pro Applikation notwendig, als bei unspezifischen Verabreichungsformen.

Es kann außerdem vorgesehen sein, dass die Aerosolerzeugungseinrichtung eine Chip-Technologie wie z.B. Bluetooth® aufweist, um etwa die Einhaltung und Compliance der Patienten zu überwachen.

Gemäß einer vorteilhaften Weiterentwicklung kann außerdem vorgesehen sein, dass die Membran an einen piezoelektrischen Kristall oder Wandler gekoppelt ist, der die Membran in Vibration versetzt. Durch diese Ausgestaltung kann die Membran zur Vibration gebracht werden, wobei eine Vibrationsfrequenz von 10 kHz bis 150 kHz bevorzugt wird. Durch die Vibration bewegt sich die Membran einige Mikrometer auf und ab und wirkt somit wie eine "Mikropumpe", indem sie die Antisense-Molekül-haltige Zusammensetzung als Aerosol in Richtung des Ausgangs der Aerosolerzeugungseinrichtung presst.

Es kann erfindungsgemäß vorgesehen sein, dass die Durchgangsöffnungen konisch ausgebildet sind, wobei der Durchmesser je einer konisch ausgebildeten Durchgangsöffnung in Richtung des Bereichs der Durchgangsöffnung, an dem das Aerosol aus der Durchgangsöffnung austritt, abnimmt. Diese Ausgestaltung sorgt für eine möglichst und einheitliche Größe der Tröpfchen im Aerosol.

Es ist auch von Vorteil, wenn die Perforation der Membran wenigstens zwei Gruppen von Durchgangsöffnungen aufweist.

Als besonders bevorzugte Variante ist bei der Verwendung einer Aerosolerzeugungseinrichtung vorgesehen, dass die Membran einen Durchmesser von 1 mm bis 8 mm aufweist, wobei insbesondere eine Membran mit einem Durchmesser von 6 mm bis 8 mm vorgesehen ist. Eine weitere vorteilhafte Ausführungsform sieht vor, dass die Membran aus einem Material hergestellt ist, das Silizium und/oder mindestens ein Metall umfasst, wobei das Metall insbesondere ein Element aus der Gruppe Edelstahl, Nickel, Palladium, Kobalt, rostfreier Stahl ist und/oder eine Legierung von mindestens zwei der Elemente ist. Dabei wird eine aus rostfreiem Stahl oder aus Nickel hergestellte Membran besonders bevorzugt. Darüber hinaus kann die perforierte Membran der Aerosolerzeugungseinrichtung rund, oval oder eckig ausgebildet sein. Die Verwendung einer Membran aus einem derartigen Material hat eine hohe Stabilität der Membran zur Folge. Gleichzeitig ist eine solche Membran günstig in der Herstellung. Eine Aerosolerzeugungseinrichtung, die eine derartige Membran aufweist, ist außerdem widerstandsfähig und somit langlebig.

Es ist außerdem von Vorteil, wenn die Zusammensetzung eine Antisense-Molekül-Konzentration von unter 75 mg/ml aufweist. So lässt sie sich ideal zur inhalativen Verabreichung bei der Behandlung von Patienten einsetzen, die an einer mit chronischen Entzündung einhergehenden Atemwegserkrankung leiden. Das liegt vor allem daran, dass Zusammensetzungen mit Antisense-Molekül-Konzentrationen, die 75 mg/ml übersteigen, derart viskos sind, dass sie sich mit einer Aerosolerzeugungseinrichtung nicht oder nur schlecht als Aerosol verabreichen lassen, weil die Durchgangsöffnungen der Perforation der Membran aufgrund der hohen Viskosität der Zusammensetzung leicht verstopfen. Zusammensetzungen mit einer Antisense-Molekül-Konzentration von über 75 mg/ml zeigen außerdem einen Wirkstoffverlust von etwa 6 %. Dies liegt an der dreidimensionalen Struktur der Antisense-Moleküle, die leicht miteinander verklumpen und somit eine Polymerstruktur ausbilden. Diese Eigenschaft ist bei DNAzymen besonders gut zu beobachten.

Es ist daher besonders von Vorteil, wenn die Zusammensetzung eine Antisense-Molekül-Konzentration von 20 mg/ml bis 50 mg/ml aufweist. Zusammensetzungen mit einer Antisense-Molekül-Konzentration von 20 mg/ml bis 50 mg/ml zeigen einen optimalen Wirkungseffekt bei der erfindungsgemäßen Verwendung mit einer Aerosolerzeugungseinrichtung.

Es kann außerdem vorgesehen sein, dass die Zusammensetzung eine Viskosität aufweist, die niedriger ist als 3,5 mPa·s. Bei einer Zusammensetzung mit einer Viskosität von über 3,5 mPa·s kann eine Aerosolerzeugungseinrichtung nicht ohne Weiteres den für die Zerstäubung bzw. Verneblung der Zusammensetzung nötigen Druck erzeugen. Das führt wiederum dazu, dass die Durchgangsöffnungen bzw. Löcher der Membran verstopfen oder verschmieren. Dieses Risiko wird durch eine Zusammensetzung, deren Antisense-Molekül-Konzentration niedriger ist als 75 mg/ml, signifikant minimiert. Eine Zusammensetzung mit einer derartigen Viskosität ist mittels der erfindungsgemäßen Verwendung problemlos und ohne signifikanten Wirkstoffverlust inhalativ applizierbar.

Als bevorzugte Variante ist bei der erfindungsgemäßen Verwendung einer Aerosolerzeugungseinrichtung vorgesehen, dass die Gruppe von Durchgangsöffnungen maximal 200 Durchgangsöffnungen pro mm² der Membran umfasst. Als besonders bevorzugte Variante ist vorgesehen, dass die Gruppe von Durchgangsöffnungen maximal 50 Durchgangsöffnungen pro mm² der Membran umfasst. Die Limitierung der Lochdichte auf 200 bzw. sogar auf 50 Durchgangsöffnungen pro mm² verstärkt den vorteilhaften Effekt der Lochdichte, wonach ein Verschmelzen, Aggregieren oder Vereinigen der Tröpfchen nach deren Austritt aus den Durchgangsöffnungen verhindert wird. Die erfindungsgemäße Verwendung von Aerosolerzeugungseinrichtungen mit Membranen, die maximal 200 bzw. maximal 50 Durchgangsöffnungen pro mm² umfassen, ermöglicht demnach eine wirksame, einfache und sichere Verabreichungsmöglichkeit der viskosen Antisense-Molekül-haltigen Zusammensetzung durch Inhalation.

Nach einer vorteilhaften Ausgestaltung der Verwendung ist vorgesehen, dass Zusammensetzung mindestens ein Antisense-Molekül umfasst, das ausgewählt ist aus der Gruppe umfassend DNAzyme, siRNAs, asDNAs oder Ribozyme. Dabei kann insbesondere vorgesehen sein, dass das mindestens eine Antisense-Molekül die Expression von GATA-3 spezifisch herunterreguliert oder die Expression von Tbet spezifisch herunterreguliert. Vorzugsweise handelt es sich bei den Antisense-Molekülen um DNAzyme. Dabei binden und spalten die spezifischen Antisense-Molekül *in vivo* die mRNA der Transkriptionsfaktoren GATA-3 und/oder Tbet, deren Proteine wiederum zentrale Schlüsselmoleküle für die Entstehung von Th1- bzw. Th2-abhängigen chronischen Entzündungserkrankungen darstellen. Durch diese Spaltung der mRNA können die mRNAs nicht mehr in funktionale Proteine translatiert werden. Folglich wird das GATA-3- bzw. Tbet-Proteinniveau signifikant minimiert.

Nach einer bevorzugten Ausführungsvariante ist vorgesehen, dass das mindestens eine Antisense-Molekül ein DNAzym ist. Bevorzugterweise ist das DNAzym ausgewählt aus einer Gruppe umfassend die DNAzyme hdg1 bis hdg70 oder aus einer Gruppe umfassend die DNAzyme td1 bis td78. Dabei ist insbesondere vorgesehen, dass das DNAzym die Sequenz hdg40 (GTGGATGGAggctagctacaacgaGTCTTGGA) aufweist. Eine derartige Sequenz zeigt eine besonders hohe Enzymaktivität und spaltet GATA-3-mRNA mit einer hohen Spezifität sowie mit hoher Effizienz. Folglich eignet sich ein DNAzym mit der Sequenz hgd40 (SEQ ID No. 40) hervorragend zur gezielten und effektiven Behandlung von Atemwegserkrankungen, die Th2-abhängig sind, die also mit einem erhöhten GATA-3-Spiegel korrespondieren. Gemäß einer vorteilhaften Weiterbildung kann die Zusammensetzung mindestens einen Nuklease-Inhibitor aufweisen, wobei der mindestens eine Nuklease-Inhibitor insbesondere Desoxyribonukleasen spezifisch inaktiviert, also ein DNase-lnhibitor ist. Durch einen derartigen Nuklease-Inhibitor wird das mindestens eine DNAzym vor dem enzymatischen Abbau durch DNasen geschützt.

Ein Beispiel einer Antisense-Molekül-haltigen Zusammensetzung ist in der folgenden Tabelle dargestellt:

| **Substanz** | **[% w/w]** |
|---|---|
| Antisense-Molekül (DNAzym) | 0,01 -0,75 |
| Nuklease-Inhibitor (z.B. DNase-lnhibitor) | variabel |

Weiterhin ist es möglich, dass die Zusammensetzung mindestens ein Salz und/oder mindestens ein Kation umfasst. Eine derartige Zusammensetzung eignet sich auf vorteilhafte Art und Weise zur Anwendung bei der Therapie von Patienten, die an einer mit chronischen Entzündungen einhergehenden Atemwegserkrankung leiden, weil die Zusammensetzung dadurch ein physiologisch günstiges Milieu aufweist und somit für Patienten leicht verträglich ist. Dabei ist insbesondere eine Phosphatgepufferte Salzlösung (PBS) vorgesehen.

Alternativ werden aber auch andere gepufferte Lösungen eingesetzt, in denen Nukleinsäuren in physiologisch günstigem Milieu gelöst vorliegen, wie zum Beispiel TE-Puffer. Entsprechend ist ein anorganischer und/oder organischer Zusatz vorgesehen, wobei der Zusatz bevorzugterweise ausgewählt ist aus der Gruppe umfassend die Stoffe Natriumchlorid (NaCl), Kaliumchlorid (KCl), Dinatriumhydrogenphosphat (Na₂HPO4), Dinatriumhydrogenphosphat Dihydrat, Kaliumdihydrogenphosphat (KH₂PO4), TRIS und EDTA. Das Kation kann dabei ausgewählt sein aus der Gruppe umfassend Na, Mg, K, Li, Ca, Fe, Cu und Ag. Alternativ ist auch ein organisches Kation vorgesehen, wie zum Beispiel Mg(N(SO₂CF₃)₂)₂ oder Mg(OSO₂CF₃)₂. Alternativ wird ein bivalentes Kation verwendet. Eine derartig gepufferte Zusammensetzung eignet sich besonders zum Schutz der Antisense-Moleküle, weil das Antisense-Molekül durch den anorganischen und/oder organischen Zusatz stabilisiert und vor enzymatischem Abbau geschützt wird. Darüber hinaus ermöglicht das Salz eine gute Aufnahme der Zusammensetzung in die Zielzellen. Die bivalenten Kationen können als Co-Faktoren der Antisense-Moleküle die Antisense-Molekül-Aktivität steigern, da die Antisense-Moleküle eine von bivalenten Kationen (vorzugsweise Mg²⁺) abhängige katalytische Domäne aufweisen. Somit wirken bivalente Kationen als "Enhancer" oder Verstärker. Nach einer vorteilhaften Weiterentwicklung kann die Zusammensetzung mindestens einen anorganischen und/oder organischen Zusatz und/oder einen Lösungsvermittler und/oder einen Konservierungsstoff umfassen. Der Lösungsvermittler dient dabei vor allem der Komplexbildung. Darüber hinaus verbessert er die Lösemitteleigenschaften der Zusammensetzung. Als Lösungsvermittler können bevorzugterweise Glycerolderivate und/oder Polyethylenglycole oder auch Lecithine vorgesehen sein. Der Konservierungsstoff kann zum Beispiel Paraben sein.

Mögliche zusätzliche Komponenten der Antisense-Molekül-haltigen Zusammensetzung sind:

| **Substanz** | **[% w/w]** |
|---|---|
| Salz und oder ein Kation (z.B. Na, Mg, K, Li, Ca, Fe, Cu, Ag, HPO₄^{2+,} H₂PO⁴⁻) | variabel |
| EDTA | variabel |
| TRIS | variabel |
| Lösungsvermittler (z.B. Glycerolderivate, Polyethylenglykole, Lecithine) | variabel |
| Konservierungsstoff (z.B. Paraben) | variabel |

Es können außerdem Zusätze vorgesehen sein, die beispielsweise eine Veränderung der Oberflächenspannung der Zusammensetzung oder eine Senkung der Viskosität der Zusammensetzung bewirken.

Die oben geschilderten Vorteile und Wirkungen ergeben sich insbesondere bei der Verwendung einer Aerosolerzeugungseinrichtung zur Erzeugung eines Aerosols einer Zusammensetzung zur inhalativen Verabreichung bei der Behandlung eines Patienten, der an einer mit chronischen Entzündungen einhergehenden Atemwegserkrankung leidet.

Weitere Merkmale, Einzelheiten und Vorteile der Erfindung ergeben sich aus dem Wortlaut der Ansprüche sowie aus der folgenden Beschreibung von Ausführungsbeispielen und Figuren.

### Ausführungsbeispiele

Die nachfolgende Tabelle 1 zeigt die GATA-3 spezifischen DNAzyme hgd1-70 mit den Sequenz-IDs gemäß Sequenzprotokoll:

| **Name** | **Sequenz** |
|---|---|
| hgd1 | 5'-TCGGTCAGAggctagctacaacgaTGCGTTGCT-3' |
| hgd2 | 5'-GGCGTACGAggctagctacaacgaCTGCTCGGT-3' |
| hgd3 | 5'-GGCGGCGTAggctagctacaacgaGACCTGCTC-3' |
| hgd4 | 5'-CTCGGGTCAggctagctacaacgaCTGGGTAGC-3' |
| hgd5 | 5'-TCCTCTGCAggctagctacaacgaCGGGGTCCT-3' |
| hgd6 | 5'-ACTCTGCAAggctagctacaacgaTCTGCGAGC-3' |
| hgd7 | 5'-GGGCGACGAggctagctacaacgaTCTGCAATT-3' |
| hgd8 | 5'-AAGGGGCGAggctagctacaacgaGACTCTGCA-3' |
| hgd9 | 5'-AAAACGGGAggctagctacaacgaCAGGTTGTA-3' |
| hgd10 | 5'-AGAATAAAAggctagctacaacgaGGGACCAGG-3' |
| hgd11 | 5'-ATGGCAGAAggctagctacaacgaAAAACGGGA-3' |
| hgd12 | 5'-AACTGGGTAggctagctacaacgaGGCAGAATA-3' |
| hgd13 | 5'-ATCCAAAAAggctagctacaacgaTGGGTATGG-3' |
| hgd14 | 5'-AGGGGAAGAggctagctacaacgaAAAAATCCA-3' |
| hgd15 | 5'-TTTTAAAAAggctagctacaacgaTATCTTGGA-3' |
| hgd16 | 5'-GTGGGGGGAggctagctacaacgaGGGAAGGCT-3' |
| hgd17 | 5'-GTTGAATGAggctagctacaacgaTTGCTTTCG-3' |
| hgd18 | 5'-GTCGTTGAAggctagctacaacgaGATTTGCTT-3' |
| hgd19 | 5'-GGCCCGGAAggctagctacaacgaCCGCGCGCG-3' |
| hgd20 | 5'-TCACCTCCAggctagctacaacgaGGCCTCGGC-3' |
| hgd21 | 5'-CCGCCGTCAggctagctacaacgaCTCCATGGC-3' |
| hgd22 | 5'-GGTGGCTCAggctagctacaacgaCCAGCGCGG-3' |
| hgd23 | 5'-CGTTGAGCAggctagctacaacgaGGCGGGGTG-3' |
| hgd24 | 5'-CCGCGTCCAggctagctacaacgaGTAGGAGTG-3' |
| hgd25 | 5'-CAGCGGGTAggctagctacaacgaTGCGCCGCG-3' |
| hgd26 | 5'-GCACATCCAggctagctacaacgaCTCCTCCGG-3' |
| hgd27 | 5'-AAAAGCACAggctagctacaacgaCCACCTCCT-3' |
| hgd28 | 5'-TAAAAAGCAggctagctacaacgaATCCACCTC-3' |
| hgd29 | 5'-GACCGTCGAggctagctacaacgaGTTAAAAAG-3' |
| hgd30 | 5'-TTGCCTTGAggctagctacaacgaCGTCGATGT-3' |
| hgd31 | 5'-AGGGCGGGAggctagctacaacgaGTGGTTGCC-3' |
| hgd32 | 5'-TGGCCCTGAggctagctacaacgaCGAGTTTCC-3' |
| hgd33 | 5'-ACCTCTGCAggctagctacaacgaCGTGGCCCT-3' |
| hgd34 | 5'-CGGAGGGTAggctagctacaacgaCTCTGCACC-3' |
| hgd35 | 5'-GGCGGCACAggctagctacaacgaCTGGCTCCC-3' |
| hgd36 | 5'-CGGGCGGCAggctagctacaacgaACCTGGCTC-3' |
| hgd37 | 5'-AGGGATCCAggctagctacaacgaGAAGCAGAG-3' |
| hgd38 | 5'-GGGTAGGGAggctagctacaacgaCCATGAAGC-3' |
| hgd39 | 5'-GGGCTGAGAggctagctacaacgaTCCAGGGGG-3' |
| hgd40 | 5'-GTGGATGGAggctagctacaacgaGT CTTGGAG-3' |
| hgd41 | 5'-CGTGGTGGAggctagctacaacgaGGACGTCTT-3' |
| hgd42 | 5'-GGGGGTAGAggctagctacaacgaGGAGAGGGG-3' |
| hgd43 | 5'-GGAGGAGGAggctagctacaacgaGAGGCCGGG-3' |
| hgd44 | 5'-GCCCCCCGAggctagctacaacgaAAGGAGGAG-3' |
| hgd45 | 5'-CCGGGGAGAggctagctacaacgaGTCCTTCGG-3' |
| hgd46 | 5'-GGACAGCGAggctagctacaacgaGGGTCCGGG-3' |
| hgd47 | 5'-TGGGGTGGAggctagctacaacgaAGCGATGGG-3' |
| hgd48 | 5'-CTTGAGGCAggctagctacaacgaTCTTTCTCG-3' |
| hgd49 | 5'-CACCTGGTAggctagctacaacgaTTGAGGCAC-3' |
| hgd50 | 5'-GCAGGGGCAggctagctacaacgaCTGGTACTT-3' |
| hgd51 | 5'-CCAGCTTCAggctagctacaacgaGCTGTCGGG-3' |
| hgd52 | 5'-GTGGGACGAggctagctacaacgaTCCAGCTTC-3' |
| hgd53 | 5'-GGAGTGGGAggctagctacaacgaGACTCCAGC-3' |
| hgd54 | 5'-ATGCTGCCAggctagctacaacgaGGGAGTGGG-3' |
| hgd55 | 5'-GGGCGGTCAggctagctacaacgaGCTGCCACG-3' |
| hgd56 | 5'-GAGGCTCCAggctagctacaacgaCCAGGGCGG-3' |
| hgd57 | 5'-GTGGGTCGAggctagctacaacgaGAGGAGGCT-3' |
| hgd58 | 5'-AGGTGGTGAggctagctacaacgaGGGGTGGTG-3' |
| hgd59 | 5'-ACTCGGGCAggctagctacaacgaGTAGGGCGG-3' |
| hgd60 | 5'-GGAGCTGTAggctagctacaacgaTCGGGCACG-3' |
| hgd61 | 5'-GGACTTGCAggctagctacaacgaCCGAAGCCG-3' |
| hgd62 | 5'-GGGCCTGGAggctagctacaacgaTTGCATCCG-3' |
| hgd63 | 5'-TGTGCTGGAggctagctacaacgaCGGGCCTTG-3' |
| hgd64 | 5'-GTTCACACAggctagctacaacgaTCCCTGCCT-3' |
| hgd65 | 5'-CAGTTCACAggctagctacaacgaACTCCCTGC-3' |
| hgd66 | 5'-CACAGTTCAggctagctacaacgaACACTCCCT-3' |
| hgd67 | 5'-GTTGCCCCAggctagctacaacgaAGTTCACAC-3' |
| hgd68 | 5'-TCGCCGCCAggctagctacaacgaAGTGGGGTC-3' |
| hgd69 | 5'-CCCGTGCCAggctagctacaacgaCTCGCCGCC-3' |
| hgd70 | 5'-GGCGTTGCAggctagctacaacgaAGGTAGTGT-3' |

Die nachfolgende Tabelle 2 zeigt die Tbet-spezifischen DNAzyme td1-78 mit ihren Sequenz-IDs gemäß Sequenzprotokoll:

| **Name** | **Sequenz** |
|---|---|
| td1 | 5'-TGGCTTCTAggctagctacaacgaGCCCTCGTC-3' |
| td2 | 5'-GGGCTCTGAggctagctacaacgaGCCTGGCTT-3' |
| td3 | 5'-GGGACCCCAggctagctacaacgaCGGAGCCCG-3' |
| td4 | 5'-GGTGGGGGAggctagctacaacgaCCCACCGGA-3' |
| td5 | 5'-GGCGGGGGAggctagctacaacgaCCGAGGGCC-3' |
| td6 | 5'-GGGCTGGGAggctagctacaacgaGGGCAGGGA-3' |
| td7 | 5'-CGTCGAGGAggctagctacaacgaCCGCCCCTC-3' |
| td8 | 5'-GGGCTGGCAggctagctacaacgaCTTCCCGTA-3' |
| td9 | 5'-CGATGCCCAggctagctacaacgaCCGGGGCGG-3' |
| td10 | 5'-GCTCCACGAggctagctacaacgaGCCCATCCG-3' |
| td11 | 5'-CCGGCTCCAggctagctacaacgaGATGCCCAT-3' |
| td12 | 5'-TCTCCGCAAggctagctacaacgaCCGGCTCCA-3' |
| td13 | 5'-CCGTCAGCAggctagctacaacgaGTCTCCGCA-3' |
| td14 | 5'-TCCCCGGCAggctagctacaacgaCGGCTCGGT-3' |
| td15 | 5'-CCCCCGCGAggctagctacaacgaGCTCGTCCG-3' |
| td16 | 5'-GTAGGGAGAggctagctacaacgaCCCAGGCTG-3' |
| td17 | 5'-GGGCGGGCAggctagctacaacgaCAAGGCGCC-3' |
| td18 | 5'-CGGGAAGGAggctagctacaacgaTCGCCCGCG-3' |
| td19 | 5'-TAGTCCTCAggctagctacaacgaGCGGCCCCG-3' |
| td20 | 5'-TCCCCGACAggctagctacaacgaCTCCAGTCC-3' |
| td21 | 5'-TTTCCCCGAggctagctacaacgaACCTCCAGT-3' |
| td22 | 5'-TGAGCGCGAggctagctacaacgaCCTCAGTTT-3' |
| td23 | 5'-GGACCACAAggctagctacaacgaAGGTGGTTG-3' |
| td24 | 5'-CTTGGACCAggctagctacaacgaAACAGGTGG-3' |
| td25 | 5'-AAACTTGGAggctagctacaacgaCACAACAGG-3' |
| td26 | 5'-CTGATTAAAggctagctacaacgaTTGGACCAC-3' |
| td27 | 5'-TGGTGCTGAggctagctacaacgaTAAACTTGG-3' |
| td28 | 5'-TGATGATCAggctagctacaacgaCTCTGTCTG-3' |
| td29 | 5'-TGGTGATGAggctagctacaacgaCATCTCTGT-3' |
| td30 | 5'-GCTTGGTGAggctagctacaacgaGATCATCTC-3' |
| td31 | 5'-ATGGGAACAggctagctacaacgaCCGCCGTCC-3' |
| td32 | 5'-GAATGGGAAggctagctacaacgaATCCGCCGT-3' |
| td33 | 5'-TGACAGGAAggctagctacaacgaGGGAACATC-3' |
| td34 | 5'-AGTAAATGAggctagctacaacgaAGGAATGGG-3' |
| td35 | 5'-CACAGTAAAggctagctacaacgaGACAGGAAT-3' |
| td36 | 5'-GCCCGGCCAggctagctacaacgaAGTAAATGA-3' |
| td37 | 5'-CCACAAACAggctagctacaacgaCCTGTAGTG-3' |
| td38 | 5'-GTCCACAAAggctagctacaacgaATCCTGTAG-3' |
| td39 | 5'-CCACGTCCAggctagctacaacgaAAACATCCT-3' |
| td40 | 5'-CCAAGACCAggctagctacaacgaGTCCACAAA-3' |
| td41 | 5'-CCACCAAGAggctagctacaacgaCACGTCCAC-3' |
| td42 | 5'-GCTGGTCCAggctagctacaacgaCAAGACCAC-3' |
| td43 | 5'-GCTCTGGTAggctagctacaacgaCGCCAGTGG-3' |
| td44 | 5'-CTGCACCCAggctagctacaacgaTTGCCGCTC-3' |
| td45 | 5'-CACACTGCAggctagctacaacgaCCACTTGCC-3' |
| td46 | 5'-CTTTCCACAggctagctacaacgaTGCACCCAC-3' |
| td47 | 5'-GCCTTTCCAggctagctacaacgaACTGCACCC-3' |
| td48 | 5'-TTCCTGGCAggctagctacaacgaGCTGCCCTC-3' |
| td49 | 5'-GTGGACGTAggctagctacaacgaAGGCGGTTT-3' |
| td50 | 5'-CCGGGTGGAggctagctacaacgaGTACAGGCG-3' |
| td51 | 5'-CCTGGCGCAggctagctacaacgaCCAGTGCGC-3' |
| td52 | 5'-CAAATGAAAggctagctacaacgaTTCCTGGCG-3' |
| td53 | 5'-TTTCCCAAAggctagctacaacgaGAAACTTCC-3' |
| td54 | 5'-ATTGTTGGAggctagctacaacgaGCCCCCTTG-3' |
| td55 | 5'-TGGGTCACAggctagctacaacgaTGTTGGACG-3' |
| td56 | 5'-TCTGGGTCAggctagctacaacgaATTGTTGGA-3' |
| td57 | 5'-GCACAATCAggctagctacaacgaCTGGGTCAC-3' |
| td58 | 5'-GGAGCACAAggctagctacaacgaCATCTGGGT-3' |
| td59 | 5-ACTGGAGCAggctagctacaacgaAATCATCTG-3' |
| td60 | 5'-ATGGAGGGAggctagctacaacgaTGGAGCACA-3' |
| td61 | 5'-TGGTACTTAggctagctacaacgaGGAGGGACT-3' |
| td62 | 5'-GGGCTGGTAggctagctacaacgaTTATGGAGG-3' |
| td63 | 5'-TCAACGATAggctagctacaacgaGCAGCCGGG-3' |
| td64 | 5'-CCTCAACGAggctagctacaacgaATGCAGCCG-3' |
| td65 | 5'-TCACCTCAAggctagctacaacgaGATATGCAG-3' |
| td66 | 5'-CGTCGTTCAggctagctacaacgaCTCAACGAT-3' |
| td67 | 5'-GTAAAGATAggctagctacaacgaGCGTGTTGG-3' |
| td68 | 5'-AAGTAAAGAggctagctacaacgaATGCGTGTT-3' |
| td69 | 5'-GGCAATGAAggctagctacaacgaTGGGTTTCT-3' |
| td70 | 5'-TCACGGCAAggctagctacaacgaGAACTGGGT-3' |
| td71 | 5'-AGGCAGTCAggctagctacaacgaGGCAATGAA-3' |
| td72 | 5'-ATCTCGGCAggctagctacaacgaTCTGGTAGG-3' |
| td73 | 5'-GCTGAGTAAggctagctacaacgaCTCGGCATT-3' |
| td74 | 5'-TATTATCAAggctagctacaacgaTTTCAGCTG-3' |
| td75 | 5'-GGGTTATTAggctagctacaacgaCAATTTTCA-3' |
| td76 | 5'-AAGGGGTTAggctagctacaacgaTATCAATTT-3' |
| td77 | 5'-CTCCCGGAAggctagctacaacgaCCTTTGGCA-3' |
| td78 | 5'-GTACATGGAggctagctacaacgaTCAAAGTTC-3' |

Bei den vorliegend getesteten Aerosolerzeugungseinrichtungen wurde jeweils eine Zusammensetzung mit einer Antisense-Molekül-Konzentration von unter 75 mg/ml eingesetzt. Anschließend wurde die Größe der Tropfen bzw. Tröpfchen des entstandenen Aerosols bestimmt.

In einer ersten Testphase wurden Aerosolerzeugungseinrichtungen mit einer perforierten Membran ohne piezoelektrischen Wandler getestet. Mit diesen Aerosolerzeugungseinrichtungen konnte eine Antisense-Molekül-haltige Zusammensetzung zur Behandlung eines an einer mit chronischen Entzündungen einhergehenden Atemwegserkrankung leidenden Patienten nicht vernebelt werden.

Dabei wurde überraschenderweise herausgefunden, dass durch die Verwendung einer Zusammensetzung mit einer Antisense-Molekül-Konzentration von unter 75 mg/ml, zusammen mit einer Aerosolerzeugungseinrichtung mit vibrierenden Membranen, wobei die Durchgangsöffnungen der Membranen einen Durchmesser von mindestens 3 µm bis höchstens 5 µm und die Membranen einen Durchmesser von 6 mm bis 8 mm aufweisen, ein Aerosol mit Tröpfchen mit einem VMD MV von ca. 4 µm entstehen. Die Durchgangsöffnungen der Membranen verstopfen dabei nicht. Dieses Phänomen lässt sich insbesondere bei einer Antisense-Molekül-Konzentration zwischen 20 mg/ml und 50 mg/ml beobachten.

Es wurden Aerosolerzeugungseinrichtungen mit verschiedenen Materialien der perforierten Membran getestet: rostfreier Stahl, Edelstahl, Nickel, Palladium, Kobalt oder Legierungen davon. Diese Materialen erweisen sich als kostengünstiger für die Herstellung der Membran als zum Beispiel Silizium, Siliziumdioxid oder Siliziumnitrid.

Es wurden Aerosolerzeugungseinrichtung mit unterschiedlichen Geometrien der Durchgangsöffnungen/Löcher der Membran getestet z.B. trichterförmige Bohrungen. Dabei zeigten insbesondere zylindrische und konische geformte Durchgangsöffnungen eine sehr gute Tropfenbildung. Die Verfahren (z.B. Lithographie, Lasercutting o.ä.), mit denen diese Bohrungen in die Membran eingebracht werden, sind dem Fachmann bekannt.

### Figuren

- Fig. 1: zeigt die grafische Darstellung der Viskosität der Antisense-Molekül-haltigen Zusammensetzung in Abhängigkeit ihrer Konzentration.
- Fig. 2: zeigt Viskositätsmessergebnisse der Antisense-Molekül-haltigen Zusammensetzung in Abhängigkeit ihrer Konzentration
- Fig. 3: zeigt Messergebnisse eines FAT ("Factory Acceptance Test") von Zusammensetzungen mit unterschiedlichen Antisense-Molekül-Konzentrationen der Antisense-Molekül-haltigen Zusammensetzung
- Fig. 4: zeigt Messergebnisse eines FAT der Antisense-Molekül-haltigen Zusammensetzung mit Antisense-Molekül-Konzentrationen von 20 mg/ml bzw. 50 mg/ml.
- Fig. 5: zeigt Messergebnisse der Antisense-Molekül-haltigen Zusammensetzung, die durch verschiedene Aerosolerzeugungseinrichtungen mit Membranen unterschiedlicher Perforationen wurden.

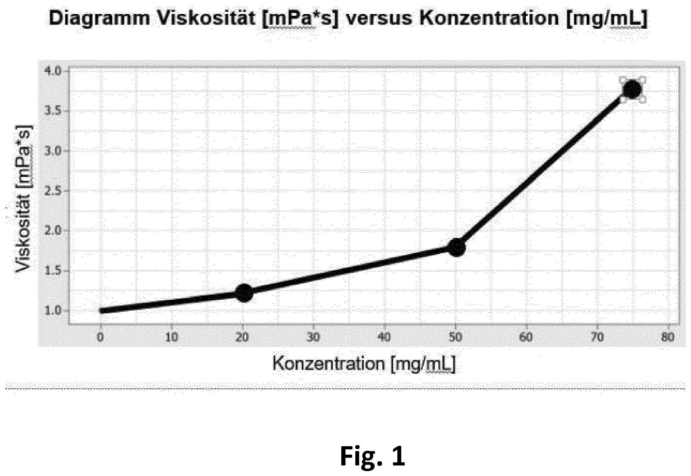

Fig. 1 zeigt in einer grafischen Darstellung die Viskosität einer Antisense-Molekül-haltigen Zusammensetzung in Abhängigkeit ihrer Konzentration. Bei dem Antisense-Molekül handelt es sich in dem gezeigten Beispiel um ein DNAzym. Das DNAzym kann dabei die Expression von GATA-3 und/oder die Expression von Tbet spezifisch herunterregulieren. Nach bevorzugter Ausführung sind vor allem Antisense-Moleküle aus einer Gruppe umfassend die DNAzyme hdg1 bis hdg70 oder aus einer Gruppe umfassend die DNAzyme td1 bis td78 vorgesehen. Bevorzugt wird das DNAzym hgd40 mit der Sequenz GTGGATGGAggctagctacaacgaGTCTTGGA eingesetzt. Hdg40 inhibiert die Expression von GATA-3 spezifisch. In Fig. 1 ist beispielhaft die Viskosität einer hgd40-haltigen Zusammensetzung graphisch dargestellt. Entlang der Y-Achse des Graphen aus Fig. 1 wird die Viskosität der Zusammensetzung in mPa·s aufgetragen; die X-Achse gibt die DNAzym-Konzentration der Zusammensetzung in mg/ml an. Aus der Darstellung ergibt sich, dass die Viskosität der DNAzym-haltigen Zusammensetzung im Wesentlichen exponentiell zunimmt. Dabei ist insbesondere ab einer Konzentration von 50 mg/ml und höher ein starker Anstieg der Viskosität zu beobachten. Bereits bei einer Konzentration von etwa 75 mg/ml ist eine Viskosität von 3,5 mPa·s zu beobachten.

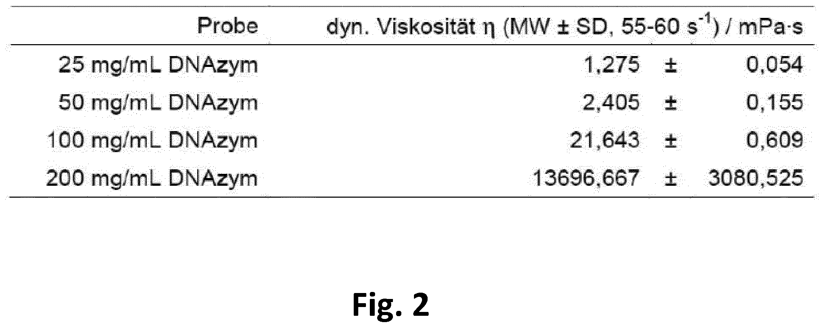

Fig. 2 zeigt die Ergebnisse einer Viskositätsmessung einer Antisense-Molekül-haltigen Zusammensetzung in Abhängigkeit ihrer Konzentration, wobei es sich bei den untersuchten Antisense-Molekülen um DNAzyme handelt. Die tabellarisch dargestellten Ergebnisse belegen den exponentiellen Anstieg der Viskosität einer Antisense-Molekül-haltigen Zusammensetzung mit einer Antisense-Molekül-Konzentration zwischen 50 mg/ml und 100 mg/ml. Die hier gezeigte Korrelation zwischen Nukleinsäurekonzentration und Viskosität ist für andere, doppelsträngige DNA-Moleküle nicht derart ausgeprägt; üblicherweise steigt die Viskosität einer Doppelstrang-DNA-haltigen Lösung im Wesentlichen linear mit steigender DNA-Konzentration. Bei einer Viskosität von über 3,5 mPa·s ist der Druck, den eine Aerosolerzeugungseinrichtung erzielen müsste, zu hoch. Das kann dazu führen, dass die Durchgangsöffnungen bzw. Löcher der Membran verstopfen oder verschmieren. Dieses Risiko wird durch eine Zusammensetzung signifikant minimiert, deren Antisense-Molekül-Konzentration niedriger ist als 75 mg/ml, weil die Viskosität einer derartigen Antisense-Molekül-haltigen Zusammensetzung unter 3,5 mPa·s liegt.

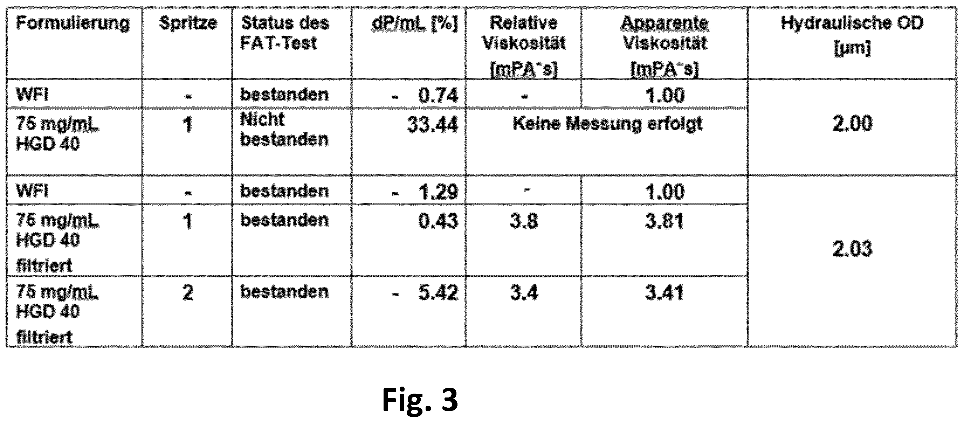

In Fig. 3 sind Messergebnisse eines FAT ("Factory Acceptance Test") von Zusammensetzungen mit unterschiedlichen Antisense-Molekül-Konzentrationen in tabellarischer Form dargestellt, wobei es sich bei den Antisense-Molekülen um DNAzyme, speziell um das DNAzym hgd40 handelt. Ein FAT ist mit einer Werksabnahme vergleichbar, in der die Anwendbarkeit eines Produkts getestet werden soll. Die Tabelle zeigt, dass sich eine Zusammensetzung mit einer Antisense-Molekül-Konzentration von über 75 mg/ml nicht mehr durch die Aerosolerzeugungseinrichtung aus dem Test vernebeln lässt (vgl. dazu Zeile 3 der Tabelle aus Fig. 3). Wird eine Zusammensetzung mit einer Antisense-Molekül-Konzentration von 75 mg/ml im Vorfeld des Testlaufs steril gefiltert, so wird die Antisense-Molekül-Konzentration auf unter 75 mg/ml herabgesenkt. Eine Zusammensetzung mit einer derartigen DNAzym-Konzentration kann mit der im Test verwendeten Aerosolerzeugungseinrichtung vernebelt werden (vgl. dazu Zeilen 5 und 6 der Tabelle aus Fig. 3). Der FAT gibt die Viskosität (dP/ml) in Prozent an. Zum Bestehen des hier durchgeführten FAT musste die Viskosität unter 5 % liegen. Die Abkürzung "WFI" in der zweiten und vierten Zeile der Tabelle aus Fig. 3 steht für "Water for Injection" und stellt die Testkontrolle dar - entsprechend korrespondieren die "WFI"-Kontrollen mit einer Viskosität von 1 dP/ml.

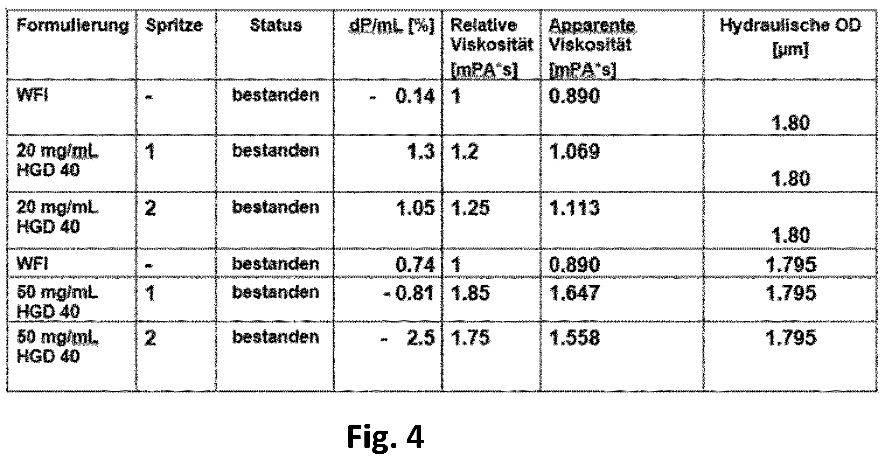

In Fig. 4 sind Messergebnisse eines FAT einer Zusammensetzung mit Antisense-Molekül-Konzentrationen von 20 mg/ml bzw. 50 mg/ml gezeigt. Dabei umfassen die hier untersuchten Antisense-Moleküle insbesondere DNAzyme. Die Ergebnisse zeigen, dass derartige Zusammensetzungen den FAT bestehen und sich mittels der erfindungsgemäßen Verwendung vernebeln lassen (vgl. dazu "bestanden" in den Zeilen 3, 4, 6 und 7 der Tabelle aus Fig. 4).

Die Messergebnisse aus den Figuren 3 und 4 zeigen also, dass eine Antisense-Molekül-haltige Zusammensetzung und hierbei insbesondere eine DNAzym-haltige Zusammensetzung mit einer DNAzym-Konzentrationen von unter 75 mg/ml mittels einer Aerosolerzeugungseinrichtung gemäß den vorliegend beschriebenen Merkmalen als Aerosol verwendet werden kann. Somit ist die erfindungsgemäße Verwendung ideal zur Behandlung eines an einer mit chronischen Entzündungen einhergehenden Atemwegserkrankung leidenden Patienten geeignet. Dabei werden bevorzugterweise Antisense-Molekül- bzw. DNAzym-Konzentrationen von 20 mg/ml bis 50 mg/ml eingesetzt.

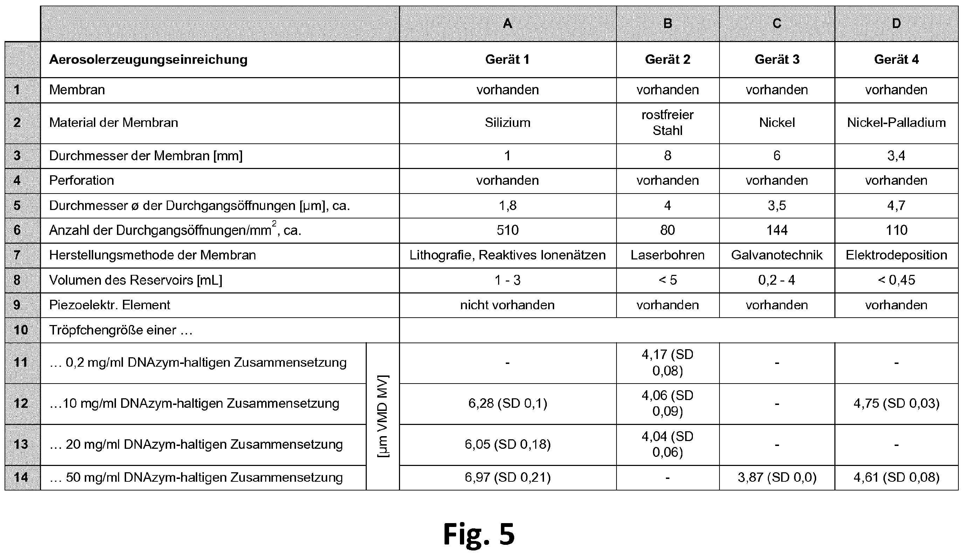

Fig. 5 zeigt Messergebnisse einiger der getesteten Aerosolerzeugungseinrichtungen. Die Messergebnisse zeigen, dass Aerosolerzeugungseinrichtungen mit den erfindungsgemäßen Merkmalen optimal zur Aerosolerzeugung einer Antisense-Molekül-haltigen Zusammensetzung geeignet sind (Fig. 5 Spalten B-D). In dem in Fig. 5 gezeigten Beispiel handelt es sich bei den Antisense-Molekülen um DNAzyme. Bei den Geräten aus den Spalten B-D entsteht das Aerosol, indem die Zusammensetzung in Kontakt mit der in Vibration versetzten Membran der Aerosolerzeugungseinrichtung gebracht wird. Erfindungsgemäß weist die Membran dabei eine Perforation auf, die von mindestens einer Gruppe von Durchgangsöffnungen gebildet ist, wobei die Durchgangsöffnungen an ihrer engsten Stelle einen Durchmesser aufweisen, der zwischen 3 µm und 5 µm liegt (Fig. 5, Zellen B5, C5 und D5), und wobei die Gruppe von Durchgangsöffnungen maximal 500 Durchgangsöffnungen pro mm² der Membran umfasst (Fig. 5, Zellen B6, C6 und D6). Dabei entstehen Tröpfchen bzw. Partikel mit einer Größe von ca. 4 µm (Fig. 5, Zellen B11-B13, C14, D12 und D14), was als optimal zur Aerosolerzeugung einer Antisense-Molekül-haltigen Zusammensetzung zur Behandlung eines an einer mit chronischen Entzündungen einhergehenden Atemwegserkrankung leidenden Patienten angesehen wird. Die Ausbildung von Tröpfchen mit der bevorzugten Größe wird durch die Vibration der Membran verstärkt.

Ein Gerät, bei dem die Durchgangsöffnungen an ihrer engsten Stelle einen Durchmesser von weniger als 3 µm aufweisen (Fig. 5, Zelle 5A) oder dessen Membran mehr als 500 Durchgangsöffnungen pro mm² (Fig. 5, Zelle 6A) aufweist, hat ein Aerosol mit Tröpfchen mit einer Größe von mehr als 5 µm VMD MV zur Folge (Fig. 12A, 13A und 14A). Tröpfchen dieser Größe haben den Nachteil, dass sie aufgrund von Impaktationskräften nicht oder zumindest nur in stark verminderter Weise an ihren entsprechenden Wirkort gelangen. Demzufolge könnten die Antisense-Moleküle bzw. insbesondere die DNAzyme ihre therapierende Wirkung nicht an dem Ort entfalten, an dem die chronische Entzündung vorliegt. Grund hierfür ist unter anderem die Lochdichte: Bei einer Lochdichte von über 500 Durchgangsöffnungen pro mm² der Membran kann es dazu kommen, dass die entstehenden Tröpfchen oder Partikel unmittelbar nach Austritt aus den Durchgangsöffnungen zu größeren Partikeln verschmelzen, aggregieren oder vereinigen.

## Patentansprüche

1. Verwendung einer Aerosolerzeugungseinrichtung zur Erzeugung eines Aerosols einer Zusammensetzung, zur inhalativen Verabreichung der Zusammensetzung,
▪ wobei die Zusammensetzung mindestens ein Antisense-Molekül umfasst,
▪ wobei die Aerosolerzeugungseinrichtung eine Membran mit einer Perforation aufweist,
▪ wobei die Zusammensetzung zur Erzeugung des Aerosols mit der Membran in Kontakt gebracht wird und die Membran der Aerosolerzeugungseinrichtung in Vibration versetzt wird,
▪ wobei die Perforation von mindestens einer Gruppe von Durchgangsöffnungen gebildet ist, und
▪ wobei jede Durchgangsöffnung einer Gruppe an ihrer engsten Stelle einen Durchmesser von mindestens 3 µm und höchstens 5 µm aufweist und wobei die mindestens eine Gruppe von Durchgangsöffnungen maximal 500 Durchgangsöffnungen pro mm² der Membran umfasst.

2. Verwendung einer Aerosolerzeugungseinrichtung nach einem der vorangegangenen Ansprüche, **dadurch gekennzeichnet, dass** die Membran an ein piezoelektrisches Element aufweist, welches die Membran in Vibration versetzt.

3. Verwendung einer Aerosolerzeugungseinrichtung nach einem der vorangegangenen Ansprüche, **dadurch gekennzeichnet, dass** die Durchgangsöffnungen der Membran zylindrisch ausgebildet sind.

4. Verwendung einer Aerosolerzeugungseinrichtung nach einem der Ansprüche 1 oder 2, **dadurch gekennzeichnet, dass** die Durchgangsöffnungen der Perforation konisch ausgebildet sind, wobei der Durchmesser jeder konischen Durchgangsöffnung in Richtung des Bereichs der Perforation, an dem das Aerosol aus der Perforation austritt, abnimmt.

5. Verwendung einer Aerosolerzeugungseinrichtung nach einem der vorhergehenden Ansprüche, **dadurch gekennzeichnet, dass** die Perforation der Membran wenigstens zwei Gruppen von Durchgangsöffnungen aufweist.

6. Verwendung einer Aerosolerzeugungseinrichtung nach einem der vorangegangenen Ansprüche, **dadurch gekennzeichnet, dass** die Membran einen Durchmesser von 6 mm bis 8 mm aufweist.

7. Verwendung einer Aerosolerzeugungseinrichtung nach einem der vorangegangenen Ansprüche, **dadurch gekennzeichnet, dass** die Membran aus einem Material hergestellt ist, das mindestens ein Metall umfasst, wobei das Metall insbesondere ein Element aus der Gruppe Edelstahl, Nickel, Palladium, Kobalt, rostfreier Stahl ist und/oder eine Legierung von mindestens zwei der Elemente ist.

8. Verwendung einer Aerosolerzeugungseinrichtung nach einem der vorangegangenen Ansprüche, **dadurch gekennzeichnet, dass** die Zusammensetzung eine Antisense-Molekül-Konzentration von unter 75 mg/ml aufweist.

9. Verwendung einer Aerosolerzeugungseinrichtung nach Anspruch 8, **dadurch gekennzeichnet, dass** die Zusammensetzung eine Antisense-Molekül-Konzentration von 20 mg/ml bis 50 mg/ml aufweist.

10. Verwendung einer Aerosolerzeugungseinrichtung nach einem der vorangegangenen Ansprüche, **dadurch gekennzeichnet, dass** die Zusammensetzung eine Viskosität aufweist, die niedriger ist als 3,5 mPa·s.

11. Verwendung einer Aerosolerzeugungseinrichtung nach einem der vorangegangenen Ansprüche, **dadurch gekennzeichnet, dass** die Gruppe von Durchgangsöffnungen maximal 350 Durchgangsöffnungen pro mm² der Membran umfasst.

12. Verwendung einer Aerosolerzeugungseinrichtung nach einem der vorangegangenen Ansprüche, **dadurch gekennzeichnet, dass** die Gruppe von Durchgangsöffnungen maximal 200 Durchgangsöffnungen pro mm² der Membran umfasst.

13. Verwendung einer Aerosolerzeugungseinrichtung nach einem der vorangegangenen Ansprüche, **dadurch gekennzeichnet, dass** die Gruppe von Durchgangsöffnungen maximal 50 Durchgangsöffnungen pro mm² der Membran umfasst.

14. Verwendung einer Aerosolerzeugungseinrichtung nach einem der vorangegangenen Ansprüche, **dadurch gekennzeichnet, dass** die Zusammensetzung mindestens ein Antisense-Molekül umfasst, das ausgewählt ist aus der Gruppe umfassend DNAzyme, siRNAs, asDNAs oder Ribozyme.

15. Verwendung einer Aerosolerzeugungseinrichtung nach Anspruch 14, **dadurch gekennzeichnet, dass** die Zusammensetzung mindestens ein Antisense-Molekül umfasst, das die Expression von GATA-3 spezifisch herunterreguliert oder mindestens ein Antisense-Molekül umfasst, das die Expression von Tbet spezifisch herunterreguliert, wobei das Antisense-Molekül ausgewählt aus einer Gruppe umfassend die DNAzyme hdg1 bis hdg70 oder aus einer Gruppe umfassend die DNAzyme td1 bis td78.

16. Verwendung einer Aerosolerzeugungseinrichtung nach Anspruch 15, **dadurch gekennzeichnet, dass** das mindestens eine Antisense-Molekül ein DNAzym ist, das die Sequenz hdg40 (GTGGATGGAggctagctacaacgaGTCTTGGA) aufweist.


SEQUENCE LISTING

<110> Sterna Biologicals GmbH & Co. KG

<120> Aerosolerzeugungseinrichtung zur inhalativen Verabreichung einer Antisense-Molekül-haltigen
Zusammensetzung

<140> EP2018
<141> 2018-07-30

<160> 148

<170> BiSSAP 1.3.5

<210> 1
<211> 33
<212> DNA
<213> Homo sapiens

<400> 1
tcggtcagag gctagctaca acgatgcgtt gct 33

<210> 2
<211> 33
<212> DNA
<213> Homo sapiens

<400> 2
ggcgtacgag gctagctaca acgactgctc ggt 33

<210> 3
<211> 33
<212> DNA
<213> Homo sapiens

<400> 3
ggcggcgtag gctagctaca acgagacctg ctc 33

<210> 4
<211> 33
<212> DNA
<213> Homo sapiens

<400> 4
ctcgggtcag gctagctaca acgactgggt agc 33

<210> 5
<211> 33
<212> DNA

<213> Homo sapiens

<400> 5
tcctctgcag gctagctaca acgacggggt cct 33

<210> 6
<211> 33
<212> DNA
<213> Homo sapiens

<400> 6
actctgcaag gctagctaca acgatctgcg agc 33

<210> 7
<211> 33
<212> DNA
<213> Homo sapiens

<400> 7
gggcgacgag gctagctaca acgatctgca att 33

<210> 8
<211> 33
<212> DNA
<213> Homo sapiens

<400> 8
aaggggcgag gctagctaca acgagactct gca 33

<210> 9
<211> 33
<212> DNA
<213> Homo sapiens

<400> 9
aaaacgggag gctagctaca acgacaggtt gta 33

<210> 10
<211> 33
<212> DNA
<213> Homo sapiens

<400> 10
agaataaaag gctagctaca acgagggacc agg 33

<210> 11
<211> 33
<212> DNA
<213> Homo sapiens

<400> 11
atggcagaag gctagctaca acgaaaaacg gga 33

<210> 12
<211> 33
<212> DNA
<213> Homo sapiens

<400> 12
aactgggtag gctagctaca acgaggcaga ata 33

<210> 13
<211> 33
<212> DNA
<213> Homo sapiens

<400> 13
atccaaaaag gctagctaca acgatgggta tgg 33

<210> 14
<211> 33
<212> DNA
<213> Homo sapiens

<400> 14
aggggaagag gctagctaca acgaaaaaat cca 33

<210> 15
<211> 33
<212> DNA
<213> Homo sapiens

<400> 15
ttttaaaaag gctagctaca acgatatctt gga 33

<210> 16
<211> 33
<212> DNA
<213> Homo sapiens

<400> 16

gtggggggag gctagctaca acgagggaag gct 33

<210> 17
<211> 33
<212> DNA
<213> Homo sapiens

<400> 17
gttgaatgag gctagctaca acgattgctt tcg 33

<210> 18
<211> 33
<212> DNA
<213> Homo sapiens

<400> 18
gtcgttgaag gctagctaca acgagatttg ctt 33

<210> 19
<211> 33
<212> DNA
<213> Homo sapiens

<400> 19
ggcccggaag gctagctaca acgaccgcgc gcg 33

<210> 20
<211> 33
<212> DNA
<213> Homo sapiens

<400> 20
tcacctccag gctagctaca acgaggcctc ggc 33

<210> 21
<211> 33
<212> DNA
<213> Homo sapiens

<400> 21
ccgccgtcag gctagctaca acgactccat ggc 33

<210> 22
<211> 33
<212> DNA
<213> Homo sapiens

<400> 22
ggtggctcag gctagctaca acgaccagcg cgg                                33

<210> 23
<211> 33
<212> DNA
<213> Homo sapiens

<400> 23
cgttgagcag gctagctaca acgaggcggg gtg                                33

<210> 24
<211> 33
<212> DNA
<213> Homo sapiens

<400> 24
ccgcgtccag gctagctaca acgagtagga gtg                                33

<210> 25
<211> 33
<212> DNA
<213> Homo sapiens

<400> 25
cagcgggtag gctagctaca acgatgcgcc gcg                                33

<210> 26
<211> 33
<212> DNA
<213> Homo sapiens

<400> 26
gcacatccag gctagctaca acgactcctc cgg                                33

<210> 27
<211> 33
<212> DNA
<213> Homo sapiens

<400> 27
aaaagcacag gctagctaca acgaccacct cct                                33

<210> 28
<211> 33

<212> DNA
<213> Homo sapiens

<400> 28
taaaaagcag gctagctaca acgaatccac ctc 33

<210> 29
<211> 33
<212> DNA
<213> Homo sapiens

<400> 29
gaccgtcgag gctagctaca acgagttaaa aag 33

<210> 30
<211> 33
<212> DNA
<213> Homo sapiens

<400> 30
ttgccttgag gctagctaca acgacgtcga tgt 33

<210> 31
<211> 33
<212> DNA
<213> Homo sapiens

<400> 31
agggcgggag gctagctaca acgagtggtt gcc 33

<210> 32
<211> 33
<212> DNA
<213> Homo sapiens

<400> 32
tggccctgag gctagctaca acgacgagtt tcc 33

<210> 33
<211> 33
<212> DNA
<213> Homo sapiens

<400> 33
acctctgcag gctagctaca acgacgtggc cct 33

<210> 34
<211> 33
<212> DNA
<213> Homo sapiens

<400> 34
cggagggtag gctagctaca acgactctgc acc 33

<210> 35
<211> 33
<212> DNA
<213> Homo sapiens

<400> 35
ggcggcacag gctagctaca acgactggct ccc 33

<210> 36
<211> 33
<212> DNA
<213> Homo sapiens

<400> 36
cgggcggcag gctagctaca acgaacctgg ctc 33

<210> 37
<211> 33
<212> DNA
<213> Homo sapiens

<400> 37
agggatccag gctagctaca acgagaagca gag 33

<210> 38
<211> 33
<212> DNA
<213> Homo sapiens

<400> 38
gggtagggag gctagctaca acgaccatga agc 33

<210> 39
<211> 33
<212> DNA
<213> Homo sapiens

<400> 39

gggctgagag gctagctaca acgatccagg ggg 33

<210> 40
<211> 33
<212> DNA
<213> Homo sapiens

<400> 40
gtggatggag gctagctaca acgagtcttg gag 33

<210> 41
<211> 33
<212> DNA
<213> Homo sapiens

<400> 41
cgtggtggag gctagctaca acgaggacgt ctt 33

<210> 42
<211> 33
<212> DNA
<213> Homo sapiens

<400> 42
gggggtagag gctagctaca acgaggagag ggg 33

<210> 43
<211> 33
<212> DNA
<213> Homo sapiens

<400> 43
ggaggaggag gctagctaca acgagaggcc ggg 33

<210> 44
<211> 33
<212> DNA
<213> Homo sapiens

<400> 44
gccccccgag gctagctaca acgaaaggag gag 33

<210> 45
<211> 33
<212> DNA
<213> Homo sapiens

<400> 45
ccggggagag gctagctaca acgagtcctt cgg                    33

<210> 46
<211> 33
<212> DNA
<213> Homo sapiens

<400> 46
ggacagcgag gctagctaca acgagggtcc ggg                    33

<210> 47
<211> 33
<212> DNA
<213> Homo sapiens

<400> 47
tggggtggag gctagctaca acgaagcgat ggg                    33

<210> 48
<211> 33
<212> DNA
<213> Homo sapiens

<400> 48
cttgaggcag gctagctaca acgatctttc tcg                    33

<210> 49
<211> 33
<212> DNA
<213> Homo sapiens

<400> 49
cacctggtag gctagctaca acgattgagg cac                    33

<210> 50
<211> 33
<212> DNA
<213> Homo sapiens

<400> 50
gcaggggcag gctagctaca acgactggta ctt                    33

<210> 51
<211> 33

<212> DNA
<213> Homo sapiens

<400> 51
ccagcttcag gctagctaca acgagctgtc ggg 33

<210> 52
<211> 33
<212> DNA
<213> Homo sapiens

<400> 52
gtgggacgag gctagctaca acgatccagc ttc 33

<210> 53
<211> 33
<212> DNA
<213> Homo sapiens

<400> 53
ggagtgggag gctagctaca acgagactcc agc 33

<210> 54
<211> 33
<212> DNA
<213> Homo sapiens

<400> 54
atgctgccag gctagctaca acgagggagt ggg 33

<210> 55
<211> 33
<212> DNA
<213> Homo sapiens

<400> 55
gggcggtcag gctagctaca acgagctgcc acg 33

<210> 56
<211> 33
<212> DNA
<213> Homo sapiens

<400> 56
gaggctccag gctagctaca acgaccaggg cgg 33

<210> 57
<211> 33
<212> DNA
<213> Homo sapiens

<400> 57
gtgggtcgag gctagctaca acgagaggag gct                33

<210> 58
<211> 33
<212> DNA
<213> Homo sapiens

<400> 58
aggtggtgag gctagctaca acgaggggtg gtg                33

<210> 59
<211> 33
<212> DNA
<213> Homo sapiens

<400> 59
actcgggcag gctagctaca acgagtaggg cgg                33

<210> 60
<211> 33
<212> DNA
<213> Homo sapiens

<400> 60
ggagctgtag gctagctaca acgatcgggc acg                33

<210> 61
<211> 33
<212> DNA
<213> Homo sapiens

<400> 61
ggacttgcag gctagctaca acgaccgaag ccg                33

<210> 62
<211> 33
<212> DNA
<213> Homo sapiens

<400> 62

gggcctggag gctagctaca acgattgcat ccg 33

<210> 63
<211> 33
<212> DNA
<213> Homo sapiens

<400> 63
tgtgctggag gctagctaca acgacgggcc ttg 33

<210> 64
<211> 33
<212> DNA
<213> Homo sapiens

<400> 64
gttcacacag gctagctaca acgatccctg cct 33

<210> 65
<211> 33
<212> DNA
<213> Homo sapiens

<400> 65
cagttcacag gctagctaca acgaactccc tgc 33

<210> 66
<211> 33
<212> DNA
<213> Homo sapiens

<400> 66
cacagttcag gctagctaca acgaacactc cct 33

<210> 67
<211> 33
<212> DNA
<213> Homo sapiens

<400> 67
gttgccccag gctagctaca acgaagttca cac 33

<210> 68
<211> 33
<212> DNA
<213> Homo sapiens

<400> 68
tcgccgccag gctagctaca acgaagtggg gtc                    33

<210> 69
<211> 33
<212> DNA
<213> Homo sapiens

<400> 69
cccgtgccag gctagctaca acgactcgcc gcc                    33

<210> 70
<211> 33
<212> DNA
<213> Homo sapiens

<400> 70
ggcgttgcag gctagctaca acgaaggtag tgt                    33

<210> 71
<211> 33
<212> DNA
<213> Homo sapiens

<400> 71
tggcttctag gctagctaca acgagccctc gtc                    33

<210> 72
<211> 33
<212> DNA
<213> Homo sapiens

<400> 72
gggctctgag gctagctaca acgagcctgg ctt                    33

<210> 73
<211> 33
<212> DNA
<213> Homo sapiens

<400> 73
gggaccccag gctagctaca acgacggagc ccg                    33

<210> 74
<211> 33

<212> DNA
<213> Homo sapiens

<400> 74
ggtgggggag gctagctaca acgacccacc gga 33

<210> 75
<211> 33
<212> DNA
<213> Homo sapiens

<400> 75
ggcgggggag gctagctaca acgaccgagg gcc 33

<210> 76
<211> 33
<212> DNA
<213> Homo sapiens

<400> 76
gggctgggag gctagctaca acgagggcag gga 33

<210> 77
<211> 33
<212> DNA
<213> Homo sapiens

<400> 77
cgtcgaggag gctagctaca acgaccgccc ctc 33

<210> 78
<211> 33
<212> DNA
<213> Homo sapiens

<400> 78
gggctggcag gctagctaca acgacttccc gta 33

<210> 79
<211> 33
<212> DNA
<213> Homo sapiens

<400> 79
cgatgcccag gctagctaca acgaccgggg cgg 33

<210> 80
<211> 33
<212> DNA
<213> Homo sapiens

<400> 80
gctccacgag gctagctaca acgagcccat ccg 33

<210> 81
<211> 33
<212> DNA
<213> Homo sapiens

<400> 81
ccggctccag gctagctaca acgagatgcc cat 33

<210> 82
<211> 33
<212> DNA
<213> Homo sapiens

<400> 82
tctccgcaag gctagctaca acgaccggct cca 33

<210> 83
<211> 33
<212> DNA
<213> Homo sapiens

<400> 83
ccgtcagcag gctagctaca acgagtctcc gca 33

<210> 84
<211> 33
<212> DNA
<213> Homo sapiens

<400> 84
tccccggcag gctagctaca acgacggctc ggt 33

<210> 85
<211> 33
<212> DNA
<213> Homo sapiens

<400> 85

cccccgcgag gctagctaca acgagctcgt ccg 33

<210> 86
<211> 33
<212> DNA
<213> Homo sapiens

<400> 86
gtagggagag gctagctaca acgacccagg ctg 33

<210> 87
<211> 33
<212> DNA
<213> Homo sapiens

<400> 87
gggcgggcag gctagctaca acgacaaggc gcc 33

<210> 88
<211> 33
<212> DNA
<213> Homo sapiens

<400> 88
cgggaaggag gctagctaca acgatcgccc gcg 33

<210> 89
<211> 33
<212> DNA
<213> Homo sapiens

<400> 89
tagtcctcag gctagctaca acgagcggcc ccg 33

<210> 90
<211> 33
<212> DNA
<213> Homo sapiens

<400> 90
tccccgacag gctagctaca acgactccag tcc 33

<210> 91
<211> 33
<212> DNA
<213> Homo sapiens

<400> 91
tttccccgag gctagctaca acgaacctcc agt                    33

<210> 92
<211> 33
<212> DNA
<213> Homo sapiens

<400> 92
tgagcgcgag gctagctaca acgacctcag ttt                    33

<210> 93
<211> 33
<212> DNA
<213> Homo sapiens

<400> 93
ggaccacaag gctagctaca acgaaggtgg ttg                    33

<210> 94
<211> 33
<212> DNA
<213> Homo sapiens

<400> 94
cttggaccag gctagctaca acgaaacagg tgg                    33

<210> 95
<211> 33
<212> DNA
<213> Homo sapiens

<400> 95
aaacttggag gctagctaca acgacacaac agg                    33

<210> 96
<211> 33
<212> DNA
<213> Homo sapiens

<400> 96
ctgattaaag gctagctaca acgattggac cac                    33

<210> 97
<211> 33

<212> DNA
<213> Homo sapiens

<400> 97
tggtgctgag gctagctaca acgataaact tgg 33

<210> 98
<211> 33
<212> DNA
<213> Homo sapiens

<400> 98
tgatgatcag gctagctaca acgactctgt ctg 33

<210> 99
<211> 33
<212> DNA
<213> Homo sapiens

<400> 99
tggtgatgag gctagctaca acgacatctc tgt 33

<210> 100
<211> 33
<212> DNA
<213> Homo sapiens

<400> 100
gcttggtgag gctagctaca acgagatcat ctc 33

<210> 101
<211> 33
<212> DNA
<213> Homo sapiens

<400> 101
atgggaacag gctagctaca acgaccgccg tcc 33

<210> 102
<211> 33
<212> DNA
<213> Homo sapiens

<400> 102
gaatgggaag gctagctaca acgaatccgc cgt 33

<210> 103
<211> 33
<212> DNA
<213> Homo sapiens

<400> 103
tgacaggaag gctagctaca acgagggaac atc                33

<210> 104
<211> 33
<212> DNA
<213> Homo sapiens

<400> 104
agtaaatgag gctagctaca acgaaggaat ggg                33

<210> 105
<211> 33
<212> DNA
<213> Homo sapiens

<400> 105
cacagtaaag gctagctaca acgagacagg aat                33

<210> 106
<211> 33
<212> DNA
<213> Homo sapiens

<400> 106
gcccggccag gctagctaca acgaagtaaa tga                33

<210> 107
<211> 33
<212> DNA
<213> Homo sapiens

<400> 107
ccacaaacag gctagctaca acgacctgta gtg                33

<210> 108
<211> 33
<212> DNA
<213> Homo sapiens

<400> 108

gtccacaaag gctagctaca acgaatcctg tag                    33

<210> 109
<211> 33
<212> DNA
<213> Homo sapiens

<400> 109
ccacgtccag gctagctaca acgaaaacat cct                    33

<210> 110
<211> 33
<212> DNA
<213> Homo sapiens

<400> 110
ccaagaccag gctagctaca acgagtccac aaa                    33

<210> 111
<211> 33
<212> DNA
<213> Homo sapiens

<400> 111
ccaccaagag gctagctaca acgacacgtc cac                    33

<210> 112
<211> 33
<212> DNA
<213> Homo sapiens

<400> 112
gctggtccag gctagctaca acgacaagac cac                    33

<210> 113
<211> 33
<212> DNA
<213> Homo sapiens

<400> 113
gctctggtag gctagctaca acgacgccag tgg                    33

<210> 114
<211> 33
<212> DNA
<213> Homo sapiens

<400> 114
ctgcacccag gctagctaca acgattgccg ctc                33

<210> 115
<211> 33
<212> DNA
<213> Homo sapiens

<400> 115
cacactgcag gctagctaca acgaccactt gcc                33

<210> 116
<211> 33
<212> DNA
<213> Homo sapiens

<400> 116
ctttccacag gctagctaca acgatgcacc cac                33

<210> 117
<211> 33
<212> DNA
<213> Homo sapiens

<400> 117
gcctttccag gctagctaca acgaactgca ccc                33

<210> 118
<211> 33
<212> DNA
<213> Homo sapiens

<400> 118
ttcctggcag gctagctaca acgagctgcc ctc                33

<210> 119
<211> 33
<212> DNA
<213> Homo sapiens

<400> 119
gtggacgtag gctagctaca acgaaggcgg ttt                33

<210> 120
<211> 33

<212> DNA
<213> Homo sapiens

<400> 120
ccgggtggag gctagctaca acgagtacag gcg 33

<210> 121
<211> 33
<212> DNA
<213> Homo sapiens

<400> 121
cctggcgcag gctagctaca acgaccagtg cgc 33

<210> 122
<211> 33
<212> DNA
<213> Homo sapiens

<400> 122
caaatgaaag gctagctaca acgattcctg gcg 33

<210> 123
<211> 33
<212> DNA
<213> Homo sapiens

<400> 123
tttcccaaag gctagctaca acgagaaact tcc 33

<210> 124
<211> 33
<212> DNA
<213> Homo sapiens

<400> 124
attgttggag gctagctaca acgagccccc ttg 33

<210> 125
<211> 33
<212> DNA
<213> Homo sapiens

<400> 125
tgggtcacag gctagctaca acgatgttgg acg 33

<210> 126
<211> 33
<212> DNA
<213> Homo sapiens

<400> 126
tctgggtcag gctagctaca acgaattgtt gga                33

<210> 127
<211> 33
<212> DNA
<213> Homo sapiens

<400> 127
gcacaatcag gctagctaca acgactgggt cac                33

<210> 128
<211> 33
<212> DNA
<213> Homo sapiens

<400> 128
ggagcacaag gctagctaca acgacatctg ggt                33

<210> 129
<211> 33
<212> DNA
<213> Homo sapiens

<400> 129
actggagcag gctagctaca acgaaatcat ctg                33

<210> 130
<211> 33
<212> DNA
<213> Homo sapiens

<400> 130
atggagggag gctagctaca acgatggagc aca                33

<210> 131
<211> 33
<212> DNA
<213> Homo sapiens

<400> 131

tggtacttag gctagctaca acgaggaggg act 33

<210> 132
<211> 33
<212> DNA
<213> Homo sapiens

<400> 132
gggctggtag gctagctaca acgattatgg agg 33

<210> 133
<211> 33
<212> DNA
<213> Homo sapiens

<400> 133
tcaacgatag gctagctaca acgagcagcc ggg 33

<210> 134
<211> 33
<212> DNA
<213> Homo sapiens

<400> 134
cctcaacgag gctagctaca acgaatgcag ccg 33

<210> 135
<211> 33
<212> DNA
<213> Homo sapiens

<400> 135
tcacctcaag gctagctaca acgagatatg cag 33

<210> 136
<211> 33
<212> DNA
<213> Homo sapiens

<400> 136
cgtcgttcag gctagctaca acgactcaac gat 33

<210> 137
<211> 33
<212> DNA
<213> Homo sapiens

<400> 137
gtaaagatag gctagctaca acgagcgtgt tgg                33

<210> 138
<211> 33
<212> DNA
<213> Homo sapiens

<400> 138
aagtaaagag gctagctaca acgaatgcgt gtt                33

<210> 139
<211> 33
<212> DNA
<213> Homo sapiens

<400> 139
ggcaatgaag gctagctaca acgatgggtt tct                33

<210> 140
<211> 33
<212> DNA
<213> Homo sapiens

<400> 140
tcacggcaag gctagctaca acgagaactg ggt                33

<210> 141
<211> 33
<212> DNA
<213> Homo sapiens

<400> 141
aggcagtcag gctagctaca acgaggcaat gaa                33

<210> 142
<211> 33
<212> DNA
<213> Homo sapiens

<400> 142
atctcggcag gctagctaca acgatctggt agg                33

<210> 143
<211> 33

<212> DNA
<213> Homo sapiens

<400> 143
gctgagtaag gctagctaca acgactcggc att 33

<210> 144
<211> 33
<212> DNA
<213> Homo sapiens

<400> 144
tattatcaag gctagctaca acgatttcag ctg 33

<210> 145
<211> 33
<212> DNA
<213> Homo sapiens

<400> 145
gggttattag gctagctaca acgacaattt tca 33

<210> 146
<211> 33
<212> DNA
<213> Homo sapiens

<400> 146
aaggggttag gctagctaca acgatatcaa ttt 33

<210> 147
<211> 33
<212> DNA
<213> Homo sapiens

<400> 147
ctcccggaag gctagctaca acgacctttg gca 33

<210> 148
<211> 33
<212> DNA
<213> Homo sapiens

<400> 148
gtacatggag gctagctaca acgatcaaag ttc 33